# EUROPEAN PATENT SPECIFICATION

(11) **EP 2 892 528 B1**
(45) Date of publication and mention of the grant of the patent: **15.05.2019**
(21) Application number: 13836176.1
(22) Date of filing: 09.09.2013
(51) Int. Cl.: A61K 31/42, A61P 11/14

(54) **PHARMACEUTICAL COMPOSITIONS COMPRISING FLURBIPROFEN**
PHARMAZEUTISCHE ZUSAMMENSETZUNGEN MIT FLURBIPROFEN
COMPOSITIONS PHARMACEUTIQUES COMPRENANT DU FLURBIPROFÈNE

(30) Priority: 07.09.2012 US 201261697901 P; 07.09.2012 GB 201215988
(43) Date of publication of application: 15.07.2015
(73) Proprietor: Reckitt Benckiser Healthcare International Limited, Slough, Berkshire SL1 3UH (GB)
(72) Inventor: SHEA, Tim, Parsippany, NJ 07054 (US); SHEPHARD, Adrian, Slough Berkshire SL1 3UH (GB); SMITH, Gary, Hull Yorkshire HU8 7DS (GB); ASPLEY, Sue, Beverley Yorkshire HU17 8SP (GB); SCHACHTEL, Bernard, P., Jupiter, FL 33477 (US)
(74) Representative: O'Brien, Niall James
(86) International application number: PCT/US2013/058685
(87) International publication number: WO 2014/039939

(56) References cited:
- WO-A1-2012/044736
- GB-A- 2 423 710
- US-A- 5 025 019
- US-A- 5 322 689
- US-A1- 2003 139 437
- US-A1- 2006 241 175
- US-A1- 2010 022 608
- ANANIA A ET AL: "Farmaci antinfiammatori e reattività bronchiale [Anti-inflammatory drugs and bronchial reactivity]", MINERVA PNEUMOLOGICA, EDIZIONI MINERVA MEDICA, IT, vol. 34, no. 3, 1 January 1995 (1995-01-01), pages 113-117, XP008097001, ISSN: 0026-4954
- DALES R E ET AL: "Chronic cough responsive to ibuprofen", PHARMACOTHERAPY : THE JOURNAL OF HUMAN PHARMACOLOGY AND DRUG THERAPY, WILEY-BLACKWELL, US, vol. 12, no. 4, 1 January 1992 (1992-01-01), pages 331-333, XP008096815, ISSN: 0277-0008

## Description

### Technical field

The present invention relates to a new medical use of Non-Steroidal Anti-Inflammatory Drugs (NSAIDs). In particular the present invention relates to an NSAID, or a pharmaceutically acceptable salt thereof, for use in the topical treatment of coughs, that is to provide an anti-tussive effect e.g. to sooth a dry cough. It has been found that ibuprofen and flurbiprofen are especially suitable NSAIDs for this use.

### Background and Prior Art

Coughing can be caused by a wide variety of conditions including infection with viral respiratory pathogens. The vast majority of acute cough is caused by upper respiratory tract viral infection. Cough is a protective reflex which removes inhaled foreign bodies and excessive secretions from the respiratory tract. Treatments for dry coughs (which do not produce significant amounts of phlegm, commonly referred to as non-productive coughs) are well known and include cough medicines which comprise an anti-tussive ingredient (to provide for cough suppression) such as Dextromethorphan.

There is always a need for the product formulator developing cough treatments to be able to formulate such products with different, or new, ingredients. For example it is always of interest to formulate such products which comprise an alternative to known anti-tussives such as Dextromethorphan.

Frequently a cough is associated with a sore throat, e.g. as the result of an upper respiratory infection. It is known to treat sore throats with an NSAID. Lozenges comprising flurbiprofen for the treatment of sore throats are available in the UK under the trade name Strefen™. WO 97/18802 (The Boots Company) discloses the use of flurbiprofen in the treatment of sore throats. No disclosure or suggestion is made in this reference to treating a cough, such as a dry cough, with NSAIDs.

NSAIDs are known for a range of pharmaceutical uses, for example analgesic formulations of ibuprofen are commercially available. However, the applicant is not aware that NSAIDs have been reported to be effective for the topical treatment of coughs, in particular dry coughs.

NSAIDs have been proposed for treating medical conditions of the gums and the mouth. EP-A-0-137-668 (Upjohn) describes the use of ibuprofen and flurbiprofen for preventing or inhibiting alveolar bone resorption. EP-A-0-486-561 (Sepracor) describes the use of S(+)-flurbiprofen or Ketoprofen to treat periodontal disease (including periodontitis, gingivitis and periodontosis) and to promote bone regrowth associated with the disease. Both these documents specifically describe the treatment of the gums with these NSAIDs and do not relate to the treatment of any other part of the oral cavity, including the throat.

There are references disclosing NSAIDs for the treatment of coughs. Examples include:
- JP 2003081821 which discloses a composition comprising ibuprofen, stramonium extract and potassium guaiacosulfonate to suppress capsaicin induced cough.
- US 2010/022608 which discloses NSAIDs for the treatment of a non-productive cough as a symptom of viral and/or bacterial respiratory infections.
- Redaelli et al's paper "Efficacy of flurbiprofen in influenza treatment for an open study" Gazette Medica Italiana Archivio per le Scienze Mediche, Vol 144, No. 11, 1985, pages 579-584 discusses the effect of flurbiprofen on cough symptoms.
- Dale et al's paper "Chronic cough responsive to Ibuprofen" Pharmacotherapy, Vol 12, No. 4, 1992, pages 331-333 discusses a study where ibuprofen was found to be effective in treating idiophathic chronic cough.
- JP 2001097856 discloses an anti-tussive formulation for treating cough symptoms, a tablet comprising ibuprofen and dimemorfan phosphate was found to suppress sulphurous acid gas induced cough.
- KR 20080039695 discloses a pharmaceutical composition for treating cough comprising gamma-polyglutamic acid lycopene and an NSAID.
- JP 2002316927 discloses a composition for treating cold and cough comprising an NSAID and pseudoephedrine.
- Sperber et al in "Effects of naproxen on experimental rhinovirus colds a randomized double-blind control trial" Annals & Internal Medicine, Vol 117, No. 1, 1992, pages 37-41 discloses a study of whether naproxen alters the course of rhinovirus colds.
- McEwan at al in "The effects of Sulindac on the abnormal cough reflex associated with dry cough" Journal of Pharmacology and Experimental Therapeutics, Vol 255, No. 1, 1990, pages 161-164 discloses patients with an angiotensin converting enzyme-associated cough had a significant reduction in cough but that patients
- with an idiopathic dry, unproductive cough did not.
- US 4 783 465 discloses a composition for treating colds and allergies comprising a propionic acid NSAID and a non-sedating anti-histamine.

It is believed that none of the above references disclose the topical effect of NSAIDs in the treatment of cough.

The present invention seeks to provide an alternative way of treating coughs, in particular dry coughs, to the methods already known in the art. It would be especially beneficial if the cough treatment could be provided together with a reduction of the pain of a sore throat.

### Statement of invention

The present invention thus provides according to a first aspect an NSAID or a pharmaceutically acceptable salt thereof for use in the topical treatment of coughs on the mucous membrane of the throat of a person in need of treatment for a cough wherein the NSAID is flurbiprofen. It has been found that treatments of dry coughs for which an anti-tussive (cough suppression) effect is required are especially effective according to the present invention.

The term "topical treatment" as used herein, and the references to "topical" refer to application to internal surfaces of the throat of a patient, that is to the mucous membranes.

The words "patient" and "person" are used interchangeably herein.

It has been found that beneficial effects in the treatment of coughs (symptoms) in a person requiring such treatment can be obtained by the topical treatment of said person with an NSAID or a pharmaceutically acceptable salt thereof by providing the NSAID so that it is topically applied in the throat of the person receiving the treatment. In trials conducted by the Applicant, coughs (symptoms) in people suffering from an upper respiratory tract infection (URI) have been reported to be less troublesome/reduced following treatment with a therapeutically effective amount of an NSAID applied topically to the mucous membrane of the throat compared to when no NSAID is administered.

Furthermore the treatment of coughs (symptoms) by administering an NSAID as above allows the amelioration of the cough and its symptoms to be achieved in a safe, effective and straightforward manner. The administration of NSAIDs is well known and does not require complicated dosage regimes. The present invention also provides the further advantage that the amelioration of the cough and its symptoms can be achieved simultaneously with other benefits such as the reduction of the pain/soreness in the throat which may be present at the same time as a cough, such as a dry cough.

### Detailed description

For the avoidance of doubt, and for the sake of brevity, references herein to the 'NSAID', or to any specific NSAID includes a reference to pharmaceutically acceptable salts thereof.

The term "lozenge" as used herein includes all dosage forms where the product is formed by cooling a sugar-based or sugar alcohol based (e.g. sorbitol) molten mass containing the active material.

The term "tablet" as used herein includes unit dosage forms made from compressed powders or granules or compressed pastes.

The references herein to "unit dose" (of the NSAID) refer to the amount of the NSAID administered in a single treatment event of the person requiring treatment for a cough. For example, a lozenge comprising an NSAID which is administered to a person in need of treatment for a cough, represents a unit dose of the NSAID. If two lozenges were administered for the same treatment event this would represent two unit doses (e.g. 2 lozenges to be taken together). If the NSAID was administered as a liquid, the amount of liquid used in a single treatment event (e.g. the number of sprays administered if the liquid is in spray-form) represents the unit dose.

### (i) NSAID types

A suitable NSAID according to the present invention is flurbiprofen.

Suitably, the NSAIDS for use in the present invention typically exhibit isomerism. Suitably, all stereoisomers, diastereoisomers, enantiomers and mixtures therefore, including racemic mixtures, of the NSAIDs are embraced by the scope of the present invention.

The NSAID, or a pharmaceutically acceptable salt thereof, according to the present invention is flurbiprofen.

Flurbiprofen [2-(2-fluoro-4-biphenylyl) propionic] acid is a well- known non-steroidal anti-inflammatory drug (NSAID) which also has analgesic and antipyretic activity. The flurbiprofen molecule exists in two enantiomeric forms and the term flurbiprofen as used herein is intended to embrace the individual enantiomers and mixtures thereof in any proportion including a 1:1 mixture which is herein referred to as racemic flurbiprofen. Flurbiprofen can exist in the form of pharmaceutically acceptable salts or in the form of derivatives such as esters and such salts or esters are embraced by the term "flurbiprofen" as used herein.

Any suitable pharmaceutically acceptable salts of the NSAIDs may be used according to the present invention.

Where a pharmaceutically acceptable salt of the NSAID, e.g. flurbiprofen, is used, the amount of the salt used should be such as to provide the desired amount of the NSAID. Suitable salts include the alkali metal salts e.g. the sodium or potassium salts, alkaline earth metal salts, for example the magnesium or calcium salts, metal salts, for example aluminum salts or amino acid salts such as the lysine, arginine or amine salts, for example meglumine salts.

The NSAID salt may be in an anhydrous or hydrated form. Preferably, the NSAID salt is in a hydrated form.

### (ii) Dosage of the NSAID

The NSAID is used in a therapeutically effective amount in the topical treatment of coughs in patients displaying cough symptoms according to the invention.

Such patients are generally suffering from a URI.

It has been found that an especially good balance of efficacy, safety and cost can be achieved for the topical treatment of coughs with an amount of the NSAID in the range of from 1 to 30 mg, such as 2 to 30 mg or 2.5 to 30 mg per unit dose of the NSAID, more preferably of from 3.5 to 20 mg per unit dose and most preferably in an amount of from 5 to 15 mg per unit dose, such as 7 to 12 or 13 mg per unit dose of the NSAID, e.g. 8 to 10 mg. This is especially the case when the NSAID is flurbiprofen.

Typically the topical treatment of the person having a cough (and so seeking an anti-tussive effect) with the NSAID will occur once about every 3 to 6 hours. The exact treatment timings will depend upon the amount of the NSAID administered for the topical treatment, the severity of the cough and the reaction of the person to the treatment. Typically the topical treatment may continue for up to 3 days. A typical topical treatment regime would be for the person to be treated with the NSAID every 3-6 hours for up to 3 days with 2 or 2.5 to 30 mg of NSAID per treatment, more preferably from 2.5 or 3.5 to 20 mg, most preferably from 5 to 15 mg, such as from 7 to 12 mg, e.g. from 8 to 10 mg of the NSAID (as a unit dose) per treatment.

### (iii) NSAID containing compositions

The NSAID used in the treatment of coughs according to the present invention may be administered in any suitable form. Preferably the NSAID is administered to a person in need of treatment for a cough as a part of a composition. Most preferably that composition is in the form of a masticable or suckable solid dosage form or a liquid.

The masticable or suckable solid dosage form may be a lozenge which is intended to be sucked by the patient or a tablet, capsule, pastille or gum, for example chewing gum. Lozenges have been found to be very suitable for administering NSAIDs for use in the treatment of coughs according to the present invention. Lozenges will normally be sucked by the patient to release the NSAID and so administer the NSAID topically to the inner surface of Patient's throat for the treatment of coughs.

The solid dosage form may also be a granular product.

A preferred composition is a lozenge prepared by cooling a heated lozenge base comprising sugar, liquid glucose, the NSAID, and other excipients to form solid lozenges. If a sugar-free, or reduced sugar, lozenge is desired sweeteners such as but not limited to sugar alcohols e.g. xylltol, maltitol, sorbitol, mannitol, erythritol and isomalt may be used.

Solid dosage forms may be prepared by methods which are well known in the art and may contain other conventional ingredients such as acidity regulators, opacifiers, stabilising agents, buffering agents, flavourings, sweeteners, colouring agents and preservatives.

For example, the lozenges may be prepared by heating the lozenge base (e.g. a mixture of sugar and liquid glucose and/or sweeteners as above) under vacuum to remove excess water and the remaining components are then blended into the mixture. The resulting mixture is then drawn into a continuous cylindrical mass from which the individual lozenges are formed. The lozenges are then cooled, subjected to a visual check and packed into suitable packaging.

Masticable solid dose formulations may be made by conventional methods used to prepare chewable candy products, tablets, chewing gums or granules. For example, a chewable solid dosage form may be prepared from an extruded mixture of sugar and glucose syrup to which the NSAID has been added with optional addition of whipping agents, humectants, lubricants, flavours and/or colourings (See Pharmaceutical Dosage Forms: Tablets, Volume 1, Second Edition edited by H A Lieberman, L Lachman and J B Schwartz published in 1989).

The liquid formulations may be in the form of a thickened or un-thickened liquid or a gel. The liquid may be used in the form of a spray which is administered to the mouth, or as a liquid e.g. a mouthwash.

The liquid formulations may be prepared by any conventional method, such as dissolving or suspending the NSAID in a liquid medium which may also contain other ingredients such as stabilising agents, buffering agents, flavourings, sweeteners, colouring agents, and preservatives. For example, a spray may be prepared by dissolving water soluble components in water and non-water soluble ingredients in a co-solvent (e.g. alcohol). The two phases are then mixed and the resulting mixture filtered and placed into dispensing containers. The dispensing containers may be fitted with a metered, manually-operated spray mechanism or the dispenser may contain a pressurised propellant and be fitted with a suitable dispensing valve.

According to the present invention, the masticable or suckable solid dosage forms can be sucked or chewed by, or the liquid composition can be administered into the mouth of, the person in need of treatment for a cough to slowly release the NSAID and so provide for said topical treatment in the throat of that person. Without wishing to be bound by theory, it is believed that the NSAID then passes over the mucous membrane of the throat where some is believed to be absorbed topically. The unabsorbed NSAID is then ingested and absorbs into the blood stream and may then act systematically to provide for the treatment of cough in addition to the relief that comes from the topical application of the NSAID to the mucous membrane of the throat.

The invention will be illustrated by the following Examples which are given by way of example only.

### Example

### 1. INTRODUCTION

Upper respiratory tract infections (URTI) comprise many symptoms and signs, which include cough and sore throat. Although mostly involuntary (e.g. in response to phlegm or irritation) coughing can be controlled, particularly when it is observed (e.g. in social situations). This phenomenon is also known to occur in sociologic and clinical research in which participants alter their behavior as a result of being observed (the 'Hawthorne effect'). To circumvent this confounding feature from a study on cough we therefore sought to examine coughing in an indirect manner, as an almost surreptitious, secondary part of a study that focused on the relief of sore throat.

To test this de-Hawthornized model, patients with sore throat and dry cough were randomized to flurbiprofen 8.75 mg lozenge or placebo lozenge and observed for 2 hours. Here we report the design and results of the first use of this new cough model.

### 2. METHODS

2a. Study population - male or female adults (≥18 years) presenting with different 'cold' symptoms at a university health center in the USA. Patient characteristics were;
- symptoms of URTI on the URTI Questionnaire (URTIQ),^{1,2} including coughing.
- Recent onset of sore throat (≤4 days)
- Moderate-to-severe throat pain on a categorical Throat Pain Scale (TPS) .
- Throat symptoms associated with pharyngitis: sore throat pain, difficulty swallowing, and the sensation of a swollen throat.
- Findings of pharyngeal inflammation on the Tonsillo-Pharyngitis assessment (TPA)².
- No evidence of lower respiratory tract disease on physical examination.

### 2b. Study medications

- One sugar based flavoured lozenge containing flurbiprofen at a dosage in the lozenge of 8.75mg.
- One sugar based flavoured vehicle-containing lozenge (placebo).

### 2c. Study instruments

The patient was asked to identify all symptoms that were currently present on the URTIQ ^{1,2}. These symptoms were;
- Runny nose
- Stuffy nose
- Ear ache
- Throat tickle
- Ear fullness
- Achiness
- Drowsy
- Heartburn
- Pressure around the eyes
- Garbled speech
- Sneezing
- Sore throat
- Mouth breathing
- Crackling ears
- Post-nasal drip
- Lack of energy
- Watery eyes
- Upset stomach
- Dizzy
- Tender neck glands
- Headache
- Throat clearing
- Wheezing
- Clogged ears
- Burning ears
- Loss of appetite
- Sinus pressure
- Acid indigestion
- Feverish
- Swollen neck glands
- Head fullness
- Coughing
- Chest tightness
- Phlegm
- Sweating
- Inability to sleep
- Sinus pain
- Nausea
- Chills
Pharyngeal symptoms were measured in the 100mm visual analogue Sore Throat Pain Intensity Scale (STIPS), Difficulty Swallowing Scale (DSS), and Swollen Throat Scale (SwoTS).

### 2d. Study design

- Randomised, double blind, placebo-controlled, single centre, parallel groups study.
- Baseline assessments of URTI symptoms (on the URTIQ) and sore throat pain (on the TPS).
- Baseline assessments of the severity of the sore throat (on the STIPS), difficulty swallowing (on the DSS), and Swollen Throat (on the SwoTS).
- Patients were randomly allocated on 1:1 to suck one_sugar based lozenge containing flurbiprofen at a dosage in the lozenge of 8.75mg or one sugar based flavoured vehicle-containing lozenge (placebo).
- Post treatment assessments on the STIPS, DSS and SwoTS.
- At 2 hours, patients again provided assessments on the STIPS, DSS, SwoTS and URTIQ.

### 2e. Statistical analysis

Efficacy endpoints included;
- The percentage of patients for which coughing on the URTIQ had resolved after 2 hours was compared between treatment groups using a chi-square test.
- 2 hours post dose.
- Least square (LS) mean change from baseline in severity in of throat symptoms as assessed using the STPIS, DSS and SwoTS at 2 hours post dose. Treatment group differences were calculated using LS means. Treatments were compared using analysis of covariance (ANOVA).
Two-sided statistical tests were performed with significance determined at the 5% level.

All analyses were performed using SAS Version 9.2.

### 3. RESULTS

### 3a. Patient population

53 patients reported sore throat and coughing as URTI symptoms at baseline. Of these, 28 patients were randomized to receive flurbiprofen 8.75mg lozenge and 25 were randomized to receive the placebo lozenge. Both treatment groups had comparable demographic and clinical features as shown in table 1 below.

**Table 1; Baseline demographics and characteristics of patients with sore throat and cough**

| | **Flurbiprofen 8.75 mg (n=28)** | **Placebo (n=25)** | **Overall (n=53)** |
|---|---|---|---|
| Age, years (mean (SD)) | 20.3 (1.98) | 19.6 (1.47) | 20.0 (1.78) |
| Male/female (%) | 50.0/50.0 | 40.0/60.0 | 45.3/54.7 |

| Duration of sore throat, n (%) | | | |
|---|---|---|---|
| 1 day | 4 (14.3) | 2 (8.0) | 6 (11.3) |
| 2 days | 12 (42.9) | 10 (40.0) | 22 (41.5) |
| 3 days | 10 (35.7) | 9 (36.0) | 19 (35.8) |
| 4 days | 2 (7.1) | 4 (16.0) | 6 (11.3) |
| TPA score (mean (SD)) | 8.6 (1.62) | 9,4 (2,50) | 9.0 (2.09) |

| Pain on the TPS (% patients) | | | |
|---|---|---|---|
| Moderate | 57.1 | 64.0 | 60.4 |
| Severe | 42.9 | 36.0 | 39.6 |
| STPIS, mm (mean (SD)) | 80.4 (8.34) | 80.5 (7.39) | 80.4 (7.83) |
| DSS, mm (mean (SD)) | 77.6 (14.70) | 75.3 (11.00) | 76.5 (13.01) |
| SwoTS, mm (mean (SD)) | 79.3 (12.05) | 77.7 (13.75) | 78.5 (12.78) |

| | | | |
|---|---|---|---|
| DSS= difficulty swallowing scale. STPIS = sore throat pain intensity scale. SwoTS =swollen throat scale. TPA = tonsillo pharyngitis Assessment. TPS = Throat pain scale. | | | |

### 3b. Cough relief

In 28 patients with sore throat and cough who received one flurbiprofen 8.75 mg lozenge, 14 patients (50%) reported no coughing at 2 hours.

In 25 patients with sore throat and cough who received one placebo lozenge, one patient (4%) reported no coughing at 2 hours ( p<0.001 compared with flurbiprofen 8.75 mg lozenge).

### 3c. Relief of Pharyngeal symptoms

At 2 hours post dose, flurbiprofen-treated patients had significantly greater reduction in sore throat pain, greater improvement in difficulty swallowing and greater reduction of swollen throat compared with patients receiving placebo (all p≤0. 01).

Difference in sore throat pain (on the STIPS) 2 hours post dose in patients with sore throat and cough;
- Flurbiprofen lozenges was -28.6
- Placebo lozenges was -14.3

Difference in difficulty swallowing (on the DSS) 2 hours post dose in patients with sore throat and cough;
- Flurbiprofen lozenges was -25.4
- Placebo lozenges was -10.4

Difference in sensation of swollen tongue (on the SwoTS) 2 hours post dose in patients with sore throat and cough;
- Flurbiprofen lozenges was -27.9
- Placebo lozenges was -9.6

### 4. DISCUSSION AND CONCLUSION

This cough model was tested on patients with an URTI (i.e. no lower respiratory tract infection). Among their multiple URTI patients complained of a sore throat and dry cough.

Compared with placebo, the flurbiprofen 8.75 mg lozenge provided relief of sore throat pain, difficulty swallowing and swollen throat (all p∼0.01.).

The flurbiprofen 8.75mg lozenge also provided relief of cough, eliminating coughing in 50% of patients, compared with cough reduction in only 4% of patients who received sugar-based vehicle (placebo) lozenge (p>0.001).

Coughing was assessed at 2 hours beyond the duration of the demulcent action of the sugar-based vehicle of the lozenge identifying an anti-tussive effect associated with flurbiprofen 8.75 mg lozenge.
1. Schachtel B.P., et al. Journal of Clinical Pharmacology 2007; 47:860-870
2. Schachtel B.P., et al. Journal of Clinical Pharmacology 2010; 50:1428-1437
3. Schachtel B.P., et al.Sore Throat Pain. In Max M.B., Porthoy R.K. and Laska E.M., eds. The Design of Analgesic Clinical Trials (advances in Pain Research and Therapy: vol 18). New York. Raven Press 1991:393-406.
4. Schachtel B.P., et al. Journal of Clinical Pharmacol ther 1984; 35:273
5. Schachtel B.P., et al. Journal of Clinical Pharmacol ther 1998; 63:173

## Claims

1. An NSAID or a pharmaceutically acceptable salt thereof for use in the topical treatment of coughs on the mucous membrane of the throat of a person in need of treatment for a cough wherein the NSAID is flurbiprofen.

2. The NSAID or a pharmaceutically acceptable salt thereof for use according to Claim 1, wherein the use is for the treatment of a dry cough.

3. The NSAID or a pharmaceutically acceptable salt thereof for use according to any one of the preceding claims, wherein the NSAID is used in an amount of from 2 to 30 mg per unit dose of the NSAID.

4. The NSAID or a pharmaceutically acceptable salt thereof for use according to claim 3, wherein the NSAID is used in an amount of from 2.5 to 30 mg per unit dose of the NSAID.

5. The NSAID or a pharmaceutically acceptable salt thereof for use according to Claim 4, wherein the NSAID is used in an amount of from 5 to 15 mg per unit dose of the NSAID.

6. The NSAID or a pharmaceutically acceptable salt thereof for use according to any one of the preceding claims, wherein the NSAID is administered to the person in need of treatment for a cough as a composition in the form of a masticable or suckable solid dosage form or a liquid.

7. The NSAID or a pharmaceutically acceptable salt thereof for use according to Claim 6, wherein the solid dosage form is a lozenge or a granule.

## Patentansprüche

1. NSAR (nichtsteroidales Antirheumatikum) oder pharmazeutisch akzeptables Salz davon zur Verwendung bei der topischen Behandlung von Husten auf der Schleimhaut des Rachens einer Person, die eine Behandlung wegen eines Hustens benötigt, wobei das NSAR Flurbiprofen ist.

2. NSAR oder pharmazeutisch akzeptables Salz davon zur Verwendung nach Anspruch 1, wobei die Verwendung zur Behandlung eines trockenen Hustens ist.

3. NSAR oder pharmazeutisch akzeptables Salz davon zur Verwendung nach einem der vorhergehenden Ansprüche, wobei das NSAR in einer Menge von 2 bis 30 mg pro Einheitsdosis des NSAR verwendet wird.

4. NSAR oder pharmazeutisch akzeptables Salz davon zur Verwendung nach Anspruch 3, wobei das NSAR in einer Menge von 2,5 bis 30 mg pro Einheitsdosis des NSAR verwendet wird.

5. NSAR oder pharmazeutisch akzeptables Salz davon zur Verwendung nach Anspruch 4, wobei das NSAR in einer Menge von 5 bis 15 mg pro Einheitsdosis des NSAR verwendet wird.

6. NSAR oder pharmazeutisch akzeptables Salz davon zur Verwendung nach einem der vorhergehenden Ansprüche, wobei das NSAR der Person, die eine Behandlung wegen eines Hustens benötigt, als eine Zusammensetzung in Form einer kaubaren oder lutschbaren festen Darreichungsform oder einer Flüssigkeit verabreicht wird.

7. NSAR oder pharmazeutisch akzeptables Salz davon zur Verwendung nach Anspruch 6, wobei es sich bei der festen Darreichungsform um eine Lutschtablette oder ein Granulat handelt.

## Revendications

1. AINS ou sel pharmaceutiquement acceptable de celui-ci pour utilisation dans le traitement topique des toux sur la muqueuse de la gorge d'une personne nécessitant le traitement d'une toux, où l'AINS est le flurbiprofène.

2. AINS ou sel pharmaceutiquement acceptable de celui-ci pour utilisation selon la Revendication 1, où l'utilisation est destinée au traitement d'une toux sèche.

3. AINS ou sel pharmaceutiquement acceptable de celui-ci pour utilisation selon l'une quelconque des revendications précédentes, où l'AINS est utilisé à une teneur comprise entre 2 et 30 mg par dose unitaire de l'AINS.

4. AINS ou sel pharmaceutiquement acceptable de celui-ci pour utilisation selon la revendication 3, où l'AINS est utilisé à une teneur comprise entre 2,5 et 30 mg par dose unitaire de l'AINS.

5. AINS ou sel pharmaceutiquement acceptable de celui-ci pour utilisation selon la Revendication 4, où l'AINS est utilisé à une teneur comprise entre 5 et 15 mg par dose unitaire de l'AINS.

6. AINS ou sel pharmaceutiquement acceptable de celui-ci pour utilisation selon l'une quelconque des revendications précédentes, où l'AINS est administré à la personne nécessitant le traitement d'une toux sous forme d'une composition qui est une forme galénique solide à mastiquer ou à sucer ou un liquide.

7. AINS ou sel pharmaceutiquement acceptable de celui-ci pour utilisation selon la Revendication 6, où la forme galénique solide est une tablette ou un granule.
